Europäisches Patentamt

**European Patent Office**

Office européen des brevets

(19)

(11) Veröffentlichungsnummer : **0 009 768**
**B2**

(12) **NEUE EUROPÄISCHE PATENTSCHRIFT**

(45) Veröffentlichungstag der neuen Patentschrift :
**18.06.86**

(21) Anmeldenummer : **79103663.5**

(22) Anmeldetag : **27.09.79**

(51) Int. Cl.⁴ : **C 07 C 31/20**, C 07 C 29/132,
**C 07 C 29/136**

(54) **Verfahren zur Herstellung von 2-Methylpentandiol-(2,4).**

(30) Priorität : **02.10.78 DE 2842942**

(43) Veröffentlichungstag der Anmeldung :
**16.04.80 Patentblatt 80/08**

(45) Bekanntmachung des Hinweises auf die Patenterteilung : **09.09.81 Patentblatt 81/36**

(45) Bekanntmachung des Hinweises auf die Entscheidung
über den Einspruch : **18.06.86 Patentblatt 86/25**

(84) Benannte Vertragsstaaten :
**DE FR GB IT NL**

(56) Entgegenhaltungen :
**DE-A- 2 214 056**
**DE-A- 2 461 615**
**DE-B- 1 202 269**
**DE-B- 2 228 332**
**DE-C- 1 038 023**
**US-A- 3 932 534**
**Chemiker-Zeitung 95 (1971) 18**

(73) Patentinhaber : **BASF Aktiengesellschaft**
**Carl-Bosch-Strasse 38**
**D-6700 Ludwigshafen (DE)**

(72) Erfinder : **Broecker, Franz Josef, Dr. Dipl.-Chem.**
**Schwanthaler Allee 20**
**D-6700 Ludwigshafen (DE)**
Erfinder : **Baur, Karl Gerhard, Dr. Dipl.-Chem.**
**Van-Leyden-Strasse 17**
**D-6700 Ludwigshafen (DE)**
Erfinder : **Platz, Rolf, Dr. Dipl.-Chem**
**Hansastrasse 5**
**D-6800 Mannheim 1 (DE)**
Erfinder : **Stabenow, Joachim, Dr. Dipl.-Phys.**
**Am Feldrain 22**
**D-6940 Weinheim (DE)**

EP 0 009 768 B2

## Beschreibung

Diese Erfindung betrifft ein neues Verfahren zur Herstellung von 2-Methylpentandiol-(2,4) durch katalytische Hydrierung von Diacetonalkohol. 2-Methylpentandiol-(2,4) wird vornehmlich als Treibstoffzusatz verwendet.

Diacetonalkohol, das bekanntlich durch alkalische Kondensation von Aceton hergestellt werden kann, läßt sich durch Hydrierung in 2-Methylpentandiol-(2,4) überführen.

Da der Diacetonalkohol chemisch und thermisch sehr instabil ist, sind bei der Hydrierung besondere Maßnahmen erforderlich, um die Nebenproduktbildung möglichst gering zu halten.

Nach dem in der GB-PS 1 182 797 beschriebenen Verfahren hydriert man Diacetonalkohol mit Raney-Nickel bei Normaldruck und Temperaturen zwischen 50 und 100 °C. Wie die Beispiele zeigen, ist die Selektivität der Hydrierung unbefriedigend. So entstehen bei relativ niedrigen Temperaturen von 50 bis 80 °C bereits 19,9 % Isopropanol und Aceton aber nur 62,5 bis maximal 83 % des gewünschten 2-Methylpentandiol-(2,4). Man ersieht daraus, wie schwierig es ist, den relativ instabilen Diacetonalkohol mit hoher Selektivität ins Diol überzuführen.

Es wurde nun gefunden, daß man 2-Methylpentandiol-(2,4) durch katalytische Hydrierung von Diacetonalkohol bei erhöhter Temperatur besonders vorteilhaft herstellen kann, wenn man einen Katalysator verwendet, der aus der Verbindung der Formel $Ni_6Al_2(OH)_{16}CO_3 \cdot 4H_2O$ durch Kalzinierung erhalten wurde, und die Hydrierung bei Drücken von 1 bis 300 bar und Temperaturen von 50 bis 120 °C in zwei Rieselreaktoren durchführt, wobei der erste Reaktor mit Rückführung und der zweite Reaktor ohne Rückführung betrieben wird, und wobei das Rücklaufverhältnis im ersten Reaktor zwischen 0,900 und 0,995 liegt.

Der erfindungsgemäß verwendete Katalysator ist aus der DE-C 2 024 282 bekannt. Er wird auch in der DE-B 22 28 332 als Hydrierungskatalysator beschrieben. Ähnliche Katalysatoren, die für die Hydrierung von Ketonen geeignet sind, werden in der US-A 3 932 534 genannt, in der Diacetonalkohol als zu dehydrierendes Keton jedoch nicht erwähnt wird. Das beanspruchte Verfahren ist durch die genannte drei Patentveröffentlichungen nicht nahegelegt, da sich die durch die vorliegende Erfindung erzielte Hydrierung von Diacetonalkohol mit hoher Selektivität z. B. nicht erreichen läßt, wenn man den aus der DE-B 22 28 332 bekannten Katalysator nach der in der US-A 3 932 534 beschriebenen Methode für die Hydrierung von Diacetonalkohol heranzieht.

Der nach der Erfindung zu verwendende Katalysator wird aus der als Katalysatorvorstufe dienenden Verbindung der Formel $Ni_6Al_2(OH)_{16}CO_3 \cdot 4H_2O$ durch Kalzinieren hergestellt. Die Katalysatorvorstufe ist z. B. durch stöchiometrische Umsetzung von wäßrigen Lösungen von Nickel- und Aluminiumsalzen, zweckmäßig den Nitraten, Ausfällen im alkalischen Bereich, z. B. durch Zugabe von Alkalicarbonat, Isolierung und Trocknung herstellbar. Die Verbindung der obengenannten Formel wird dann kalziniert, wobei sie in ein Oxidgemisch übergeführt wird. Man kalziniert z. B. durch 5 bis 24-stündiges Erhitzen auf 300 bis 450 °C, vorzugsweise auf 350 bis 400 °C.

Es ist vorteilhaft, den Katalysator zu verformen, z. B. zu Tabletten, und vor der erfindungsgemäßen Hydrierung mit Wasserstoff zu reduzieren. Bei der an sich bekannten Herstellung von Pillen setzt man dem feinverteilten Katalysator zweckmäßig bis zu 2 Gew.% an Graphit als Schmiermittel zu. Die Vorhydrierung führt man z. B. durch, indem man den Katalysator, zweckmäßig nach dessen Einbau in den zu verwendenden Hydrierreaktor, z. B. 12 bis 60 Stunden bei Temperaturen von 300 bis 450 °C, vorteilhaft 350 bis 400 °C, mit Wasserstoff behandelt.

Der erfindungsgemäß zu verwendende Katalysator zeigt schon bei relativ tiefen Temperaturen und Drücken eine wesentlich höhere Aktivität und Selektivität als andere Nickelträgerkatalysatoren.

Bei der erfindungsgemäßen Hydrierung wählt man Temperaturen von 50 bis 120 °C, vorzugsweise 60 bis 100 °C, und Drucke von 1 bis 300 bar, vorzugsweise 30 bis 200 bar.

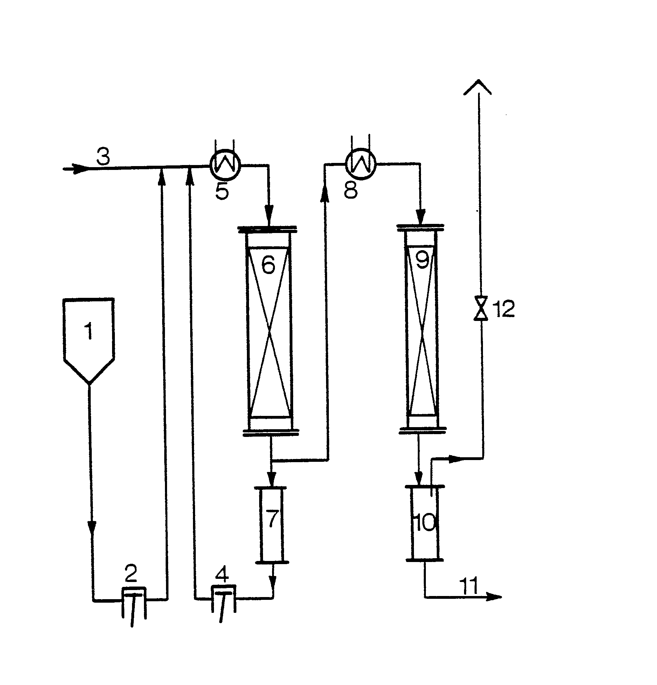

Die Hydrierung führt man, wie in der Abbildung veranschaulicht, in Rieselfahrweise unter Verwendung von zwei mit dem Katalysator beschickten Reaktoren durch.

Das zu hydrierende Ausgangsprodukt wird aus dem Behälter (1) mit einer Dosierpumpe (2) in den Hydrierkreislauf eingespeist und zwar zusammen mit Wasserstoff (3). Der Hydrierkreislauf wird mit einer Kreislaufpumpe (4) aufrechterhalten. Mit einem Wärmeaustauscher (5) wird die gewünschte Reaktoreingangstemperatur eingestellt. Die Flüssigkeit rieselt dann bei einem bestimmten $H_2$-Partialdruck über das Katalysatorbett des ersten Reaktors (6) und wird dabei hydriert. Über die nachgeschaltete Standhaltung (7) wird ein Teil der Hydrierlösung aus dem Kreislauf ausgetragen und gelangt über einen Wärmeaustauscher (8) in den zweiten Hydrierreaktor (9), der ohne Rückführung betrieben wird. Über eine Standhaltung (10) wird das Produkt ausgetragen (11) und gleichzeitig wird am Abscheider (10) über ein Ventil (12) Abgas ausgeschleust. Der erste Reaktor (6) wird mit hoher Rückführung betrieben, d. h. man hat einen großen Flüssigkeitskreislauf mit einer Querschnittsbelastung für den Katalysator im Bereich von 20 bis 60 $[m^3/m^2] \cdot h$.

Dadurch erhält man auch im ersten Reaktor (6) bei einem Umsatz von > 89 % ein geringes $\Delta T$. Der nachgeschaltete zweite Reaktor (9) wird im geraden Durchgang ohne Rückführung betrieben und liefert ein völlig durchhydriertes Produkt.

Das Rücklaufverhältnis

$$\left( \frac{\text{Gewicht der Rücklaufmenge}}{\text{Gewicht der Rücklaufmenge} + \text{Gewicht der Zulaufmenge}} \right)$$

im ersten Reaktor liegt zwischen 0,900 und 0,995, vorzugsweise zwischen 0,930 und 0,990.

Die hohe Katalysatoraktivität ermöglicht es, daß bei dem neuen Verfahren bei relativ niedrigen Temperaturen und Drucken 2-Methylpentandiol-(2,4) mit hoher Ausbeute und Selektivität erhalten wird.

### Beispiele 1 bis 3

### Herstellung des Katalysators

279,4 kg Ni(NO$_3$)$_2$ · 6H$_2$O und 120 kg Al(NO$_3$)$_3$ · 9H$_2$O werden so in Wasser gelöst, daß eine Lösung von 640 Liter erhalten wird. Man stellt nun eine zweite Lösung dadurch her, daß man 159 kg technisches Natriumbicarbonat so in Wasser löst, daß 750 Liter erhalten werden. In einem Fällungsgefäß legt man so viel Wasser vor, daß die erhaltene Mischung gut rührbar ist.

Beide Lösungen und die Wasservorlage werden getrennt auf 80 °C erhitzt. Die Wasservorlage wird dann mit der zweiten Lösung unter Rühren auf pH = 8,0 eingestellt. Durch gleichzeitigen Zulauf der beiden Lösungen in die Vorlage wird die Fällung bei 80 °C und einem pH-Wert von 8,0 durchgeführt. Nach beendeter Fällung wird noch 15 Minuten bei 80 °C nachgerührt und dann abfiltriert. Der erhaltene Niederschlag wird solange mit Wasser gewaschen, bis kein Nitrat mehr im Filtrat nachzuweisen ist. Der gewaschene Rückstand wird bei 110 °C getrocknet. Er hat die Formel Ni$_6$Al$_2$(OH)$_{16}$CO$_3$ · 4H$_2$O. Man kalziniert nun, indem man die Katalysatorvorstufe 5 bis 24 Stunden auf 350 °C erhitzt. Nach dem Abkühlen wird der Katalysator unter Zusatz von 2 Gewichtsprozent Graphit zu Pillen der Größen 3 × 3 mm verpreßt.

### Herstellung von 2-Methylpentan-diol-(2,4)

Man führt die Hydrierung in einem Rieselreaktoren mit Rückführung durch 200 ml des Katalysators werden in den Rieselreaktor eingebaut. Man behandelt den Katalysator 24 Stunden mit Wasserstoff bei 400 °C. Dann wird auf die in der Tabelle angegebene Reaktionstemperatur abgekühlt und unter gleichzeitiger Zufuhr von Wasserstoff ein Gemisch folgender Zusammensetzung eingepumpt.

| | |
|---|---|
| Diacetonalkohol | 97,92 [Gew.%] |
| 2-Methylpentandiol-(2,4) | 0,17 [Gew.%] |
| Mesityloxid | 0,38 [Gew.%] |
| Isopropanol | 0,84 [Gew.%] |
| Aceton | 0,66 [Gew.%] |

Über die Standhaltung am Ausgang des Reaktors wird das Hydrierprodukt kontinuierlich ausgetragen. Die Reaktionsbedingungen und Ergebnisse sind in der Tabelle angegeben.

### Vergleichsbeispiele a bis c

a) Man verfährt entsprechend den oben beschriebenen Beispielen, wobei man jedoch als Katalysator 200 ml eines Nickel-Silikat-Katalysators aus 70 Gew.% NiO und 30 Gew.% SiO$_2$ in Form von 3 × 3 mm Pillen verwendet. Die Reaktionsbedingungen und Ergebnisse sind in der Tabelle angegeben. Man erkennt, daß selbst bei höherer Temperatur als im Beispiel 2 der Umsatz nur 73,9 % beträgt. Die Selektivität von nur 87,5 % ist auf die vermehrte Bildung von Mesityloxid (7,4 %) zurückzuführen.

b) und c) Man verfährt entsprechend den Angaben in den oben beschriebenen Beispielen, wobei man jedoch als Katalysator 200 ml eines Kobalt-Ammoniumoxid-Katalysators aus 75 Gew.% Kobaltoxid und 25 Gew.% Aluminiumoxid in Form von 3 × 3 mm Pillen verwendet und den Katalysator vor der Umsetzung 24 Stunden bei 400 °C mit einem Gemisch aus 95 Vol.% Stickstoff und 5 Vol.% Wasserstoff reduziert. Die Reaktionsbedingungen und Ergebnisse sind in der Tabelle angegeben.

Wie man sieht, sind die Ausbeuten an 2-Methylpentandiol-(2,4) schlecht. Die Selektivität beträgt bei 60 °C nur 45,5 % und sinkt mit steigender Temperatur. Die schlechten Resultate werden durch die starke Rückspaltung des Diacetonalkohols zu Aceton bewirkt. Ein Teil des Acetons wird dann als Folgeprodukt zu Isopropanol hydriert.

(Siehe Tabelle Seite 4 f.)

Tabelle

| Bei-spiel Nr. | Tempe-ratur [°C] | Druck [bar] | Belas-tung [l/l. Kat. h] | Rücklauf-verhältnis | Analyse des Hydrierproduktes [Gew. %] | | | | | Ausbeute an 2-Methyl pentandiol-[2,4] [%] | Umsatz [%] | Selekti-vität |
|---|---|---|---|---|---|---|---|---|---|---|---|---|
| | | | | | Diaceton-alkohol | 2-Methyl-pentandiol [2,4] | Aceton | Isopro-panol | Mesithyl-oxid | | | |
| 1 | 70 | 30 | 0,5 | 0,990 | 10.4 | 86,0 | 0,3 | 1,9 | 0.8 | 86,4 | 89,3 | 96,8 |
| 2 | 90 | 100 | 1,0 | 0,980 | 10,5 | 86,9 | — | 1,4 | 1,1 | 87,2 | 89,3 | 97,6 |
| 3 | 90 | 200 | 0,5 | 0,990 | 3,3 | 93,4 | 0,1 | 1,1 | 2,0 | 93,7 | 96,7 | 96,8 |
| a | 100 | 100 | 1.0 | 0.980 | 25.8 | 65,1 | — | 1,0 | 7,4 | 64,7 | 73,9 | 87,5 |
| b | 60 | 30 | 0,5 | 0,990 | 25,4 | 34,0 | 16,3 | 22,0 | 0,4 | 33,8 | 74,3 | 45,5 |
| c | 80 | 30 | 1,5 | 0,971 | 37,4 | 20,3 | 22,6 | 16,7 | 0,3 | 20,2 | 62,2 | 32,5 |

# 0 009 768

**Patentanspruch**

Verfahren zur Herstellung von 2-Methylpentandiol-(2,4) durch katalytische Hydrierung von Diacetonalkohol bei erhöhter Temperatur, dadurch gekennzeichnet, daß man einen Katalysator verwendet, der aus der Verbindung der Formel $Ni_6Al_2(OH)_{16}CO_3 \cdot 4H_2O$ durch Kalzinierung erhalten wurde, und die Hydrierung bei Drücken von 1 bis 300 bar und Temperaturen von 50 bis 120 °C in zwei Rieselreaktoren durchführt, wobei der erste Reaktor mit Rückführung und der zweite Reaktor ohne Rückführung betrieben wird, und wobei das Rücklaufverhältnis im ersten Reaktor zwischen 0,900 und 0,995 liegt.

**Claim**

A process for the preparation of 2-methylpentane-2,4-diol by catalytically hydrogenating diacetone-alcohol at elevated temperature, characterized in that a catalyst which has been obtained by calcining a compound of the formula $Ni_6Al_2(OH)_{16}CO_3 \cdot 4H_2O$ is used, and the hydrogenation is carried out at a pressure of from 1 to 300 bar and a temperature of from 50° to 120 °C in two trickle reactors, the first reactor being operated with recycling and the second reactor without recycling, and the recycle ratio in the first reactor being from 0.900 to 0.995.

**Revendication**

Procédé de préparation du 2-méthylpentane-diol-(2,4) par hydrogénation catalytique du diacétone-alcool à chaud, caractérisé en ce que l'on utilise un catalyseur qui a été obtenu à partir du composé de formule $Ni_6Al_2(OH)_{16}CO_3 \cdot 4H_2O$ par calcination et en ce que l'on effectue l'hydrogénation à des pressions de 1 à 300 bars et des températures de 50 à 120 °C dans deux réacteurs à ruissellement, le premier réacteur opérant avec recyclage et le deuxième sans recyclage et le rapport de recyclage dans le premier réacteur se situant entre 0,900 et 0,995.